# EUROPÄISCHE PATENTSCHRIFT

(11) **EP 1 448 507 B1**
(45) Veröffentlichungstag und Bekanntmachung des Hinweises auf die Patenterteilung: **29.12.2010**
(21) Anmeldenummer: 02779567.3
(22) Anmeldetag: 21.11.2002
(51) Int. Cl.: C07C 67/54, C11C 1/10, C07C 69/58

(54) **VERFAHREN ZUR HERSTELLUNG VON TECHNISCHEN ÖLSÄUREMETHYLESTERN**
METHOD FOR PRODUCING TECHNICAL OLEIC ACID METHYL ESTERS
PROCEDE DE PRODUCTION D'ESTERS DE METHYLE D'ACIDE OLEIQUE TECHNIQUES

(30) Priorität: 30.11.2001 FR 0115503
(43) Veröffentlichungstag der Anmeldung: 25.08.2004
(73) Patentinhaber: Cognis France, S.A.S., 77310 St. Fargeau Ponthierry (FR)
(72) Erfinder: HECK, Stephan, 50259 Pulheim (DE); WINTERHOFF, Volker, 40885 Ratingen (DE); GUTSCHE, Bernhard, 40724 Hilden (DE); FIEG, Georg, 40822 Mettmann (DE); MÜLLER, Uwe, 40789 Monheim (DE); RIGAL, Jean, F-31170 Tournefeuille (FR); KAPALA, Thomas, 40882 Ratingen (DE)
(74) Vertreter: Fabry, Bernd
(86) Internationale Anmeldenummer: PCT/EP2002/013051
(87) Internationale Veröffentlichungsnummer: WO 2003/045891

(56) Entgegenhaltungen:
- EP-A- 0 460 917
- EP-A- 0 610 506
- WO-A-99/25674
- DE-A- 4 124 517
- K.-H. SCHMID: "Möglichkeiten der Fettchemie für die Schmiermittelindustrie" FETT WISSENSCHAFT TECHNOLOGIE- FAT SCIENCE TECHNOLOGY., Bd. 89, Nr. 6, 1987, Seiten 237-248, XP002232102 CONRADIN INDUSTRIEVERLAG. LEINFELDEN ECHTERDINGEN., DE ISSN: 0931-5985

## Beschreibung

### Gebiet der Erfindung

Die Erfindung befindet sich auf dem Gebiet der Oleochemie und betrifft ein Verfahren zur Herstellung von technischen Ölsäuremethylestern mit geringem Stearinsäuremethylestergehalt durch mehrfache Fraktionierung von Palmkernfettsäuremethylester.

### Stand der Technik

Ungesättigte Fettalkohole mit lodzahlen im Bereich von 80 bis 100 stellen wertvolle Rohstoffe für die Herstellung von Detergentien und Kosmetika her. Die Synthese dieser Verbindungen erfolgt durch Reduktion der entsprechenden technischen Ölsäuremethylester, die infolge ihrer natürlichen Herkunft stets Anteile gesättigter Homologe enthalten. Um ein einwandfreies Kälteverhalten zu garantieren, ist es jedoch erforderlich, dass der Gehalt an Stearinsäuremethylester in diesen Ausgangsstoffen unter 2 Gew.-% liegt. Um dies zu gewährleisten, werden die den Estern zugrundeliegenden Fettsäuren üblicherweise einer Netzmitteltrennung (Umnetzung) oder einer Lösungsmittelkristallisation (Emersol-Verfahren) unterworfen, was zum einen technisch aufwendig ist und zum anderen die einfachere Route zur Herstellung der Ester durch Umesterung der Triglyceride unmöglich macht. Alternativ besteht die Möglichkeit, eine fraktionierte Kristallisation auch auf der Stufe der Alkohole durchzuführen (WO 98/42646, Cognis), was jedoch ebenfalls mit einem zusätzlichen Verarbeitungsschritt verbunden wäre und daher unerwünscht ist.

Aus der WO 99/25674 A (Henkel KGaA) ist außerdem ein Verfahren zur Herstellung von ungesättigten Palmkernfettalkoholen durch zweifache Fraktionierung von Palmkernfettsäuremethylester. Hier fraktioniert man zunächst Palmkernfettsäuremethylester unter Erhalt einer Fraktion mit überwiegend C16-C18-Anteilen, welche in einer zweiten Fraktionierung in ein überwiegend gesättigtes Destillat und ein überwiegend ungesättigtes Sumpfprodukt fraktioniert wird. Das Sumpfprodukt wird dann unter Erhalt der Doppelbindungen zu den entsprechenden Alkoholen hydriert. Man erhält ein Hydrierprodukt mit 62,3 % Oleylalkohol.

Die Aufgabe der vorliegenden Erfindung hat somit darin bestanden, ein alternatives Verfahren zur Herstellung von Ölsäuremethylestern mit Methylstearatgehalten kleiner oder gleich 2 Gew.-% zur Verfügung zu stellen, das frei von den geschilderten Nachteilen ist. Insbesondere sollte das Verfahren ohne zusätzliche Kristallisationsschritte auskommen und kontinuierlich betrieben werden können. Vorzugsweise sollte das Verfahren Ölsäuremethylester in einer Reinheit von mindestens 75 Gew.-% liefern.

### Beschreibung der Erfindung

Gegenstand der Erfindung ist ein Verfahren zur Herstellung von technischen Ölsäuremethylestern mit Anteilen an Stearinsäuremethylester kleiner oder gleich 2 Gew.-% und Anteilen an Palmitinsäuremethylester kleiner oder gleich 5 Gew.-%. Ausgangsgemisch ist Palmkernfettsäuremethylester (PKFSME C8-18), welches zunächst fraktioniert destilliert wird, so dass sich im Kopfprodukt PKFSME C8-14 und im Sumpfprodukt PKFSME C16-18 anreichert. Gegenstand der Erfindung ist nun ein Verfahren zur weiteren Verarbeitung der Sumpffraktion PKFSME C16-18, welches sich dadurch auszeichnet, dass man
(a) PKFSME C16-18 einer ersten fraktionierten Destillation unterwirft und dabei ein kürzerkettiges Kopfprodukt PKFSME C16 und ein überwiegend längerkettiges und ungesättigtes Sumpfprodukt PKFSME C18 herstellt, und
(b) das so erhaltene erste Sumpfprodukt einer zweiten fraktionierten Destillation unterwirft und dabei ein überwiegend ungesättigtes zweites Kopfprodukt mit erhöhtem Palmitinsäuremethylestergehalt und ein ebenfalls überwiegend ungesättigtes Sumpfprodukt mit minimalem Palmitinsäuremethylestergehalt, wobei das zweite Kopfprodukt mindestens 1 Gew.-% Palmitinsäuremethylester und mindestens 60 Gew.-% Ölsäuremethylester aufweist, und
(c) das so erhaltene zweite Sumpfprodukt einer dritten fraktionierten Destillation unterwirft und dabei ein ölsäuremethylesterreiches und stearinsäuremethylesterarmes drittes Kopfprodukt und ein ölsäuremethylester- und stearinsäuremethylesterreiches Sumpfprodukt herstellt, wobei das dritte Kopfprodukt mindestens 75 Gew.-% Ölsäuremethylester und weniger als 2 Gew.-% Stearinsäuremethylester enthält, und
(d) das Zielprodukt durch Mischen des zweiten Kopfproduktes und des dritten Kopfproduktes herstellt, wobei das Zielprodukt mindestens 75 Gew.-% Ölsäuremethylester, maximal 5 Gew.-% Palmitinsäuremethylester und maximal 2 Gew.-% Stearinsäuremethylester enthält.

Das Endprodukt ist eine Mischung der Kopfprodukte aus den Stufen zwei und drei.

Überraschenderweise wurde gefunden, dass die definierte Auftrennung von Palmkernfettsäuremethylester schließlich zu einem Ölsäuremethylester führt, der einen Reinheitsgrad von mindestens 75 Gew.-% aufweist, dabei praktisch frei von Stearinsäuremethylester ist und einen Palmitinsäuremethylestergehalt von kleiner 5 Gew.-% aufweist, ohne dass dieses Ergebnis unter Einbeziehung einer Umnetztrennung bzw. einer fraktionierten Kristallisation erzielt wurde.

### Erste Fraktionierung - Abtrennung von PKFSME C16

Alle drei Fraktionierungsschritte können in an sich bekannter Weise durchgeführt werden. Die erste Stufe des Verfahrens dient im wesentlichen dazu, den Hauptteil des unerwünschten Palmitinsäuremethylesteranteil abzutrennen. Zu diesem Zweck werden die Einsatzstoffe ("Feed") auf die Siedetemperatur erhitzt und zwischen Auftriebs- und Abtriebsteil einer Fraktionierkolonne eingespeist. Die Kolonne ist vorzugsweise mit gepackten Einbauten versehen, beispielsweise Melapak der Firma Sulzer. Die erste Fraktionierung wird vorzugsweise bei einer Temperatur im Bereich von 160 bis 200 und insbesondere 180 bis 190 °C und einem verminderten Druck im Bereich von 10 bis 25 mbar durchgeführt, wobei der Druckabfall in der Kolonne üblicherweise etwa 15 mbar beträgt. Das Rücklaufverhältnis kann 2 bis 3 betragen. Auf diese Weise wird ein erstes Kopfprodukt hergestellt, welches mindestens 90 und vorzugsweise 95 bis 99 Gew.-% Palmitinsäuremethylester enthält. Gleichzeitig wird ein erstes Sumpfprodukt erhalten, welches mindestens 70 und vorzugsweise 70 bis 75 Gew.-% Ölsäuremethylester enthält. Die Prozessparameter wie z.B. das Rückflussverhältnis können variieren, weil sie von der Feed-Zusammensetzung abhängig sind.

### Zweite Fraktionierung

Die zweite Stufe des Verfahrens dient im wesentlichen dazu, die verbliebenen Anteile an Palmitinsäuremethylester zu entfermen und bereits ungesättigte PKFSME C18 im Kopfprodukt anzureichern. Die zweite Fraktionierung wird vorzugsweise bei einer Temperatur im Bereich von 200 bis 230 und insbesondere 210 bis 220 °C und einem verminderten Druck im Bereich von 10 bis 25 mbar durchgeführt, wobei der Druckabfall in der Kolonne üblicherweise etwa 15 mbar beträgt. Das Rücklaufverhältnis kann 8 bis 12 betragen. Auf diese Weise wird ein zweites Kopfprodukt hergestellt, welches noch mindestens 1 und vorzugsweise 10 bis 15 Gew.-% Palmitinsäuremethylester und mindestens 60 Gew.-%, vorzugsweise 65 bis 80 Gew.-% Ölsäuremethylester enthält. Gleichzeitig wird ein zweites Sumpfprodukt erhalten, welches höchstens noch 1 Gew.-% Palmitinsäuremethylester und mindestens 70, vorzugsweise 70 bis 75 Gew.-% Ölsäuremethylester aufweist.

### Dritte Fraktionierung

Die dritte und letzte Stufe des Verfahrens dient dazu, den Stearinsäuremethylester praktisch quantitativ abzutrennen, d.h. im Sumpf zu belassen. Die dritte Fraktionierung wird vorzugsweise bei einer Temperatur im Bereich von 200 bis 230 und insbesondere 210 bis 220 °C und einem verminderten Druck im Bereich von 10 bis 25 mbar durchgeführt, wobei der Druckabfall in der Kolonne üblicherweise etwa 15 mbar beträgt. Das Rücklaufverhältnis kann 5 bis 10 betragen. Auf diese Weise wird ein drittes Kopfprodukt hergestellt, welches mindestens 75, vorzugsweise 80 bis 85 Gew.-% Ölsäuremethylester und weniger als 2 Gew.-% Stearinsäuremethylester enthält. ) Die beiden Kopfprodukte aus den Stufen 2 und 3 stellen das Zielprodukt dar. Das Kopfprodukt aus Stufe 1 und das Sumpfprodukt aus Stufe 3 kann entweder in den Kreislauf zurückgefahren, um die Ausbeute zu erhöhen oder zu anderen Zwecken verwendet werden.

### Beispiele

**Beispiel 1.** Palmkernfettsäuremethylesterfraktion (PKFSME 16-18) wurde mit einem Durchsatz von 2000 kg/h auf 180 °C erhitzt und kontinuierlich zwischen Auftriebs- und Abtriebsteil einer ersten Fraktionierkolonne mit gepackten Einbauten aufgegeben. Bei einem Kopfvakuum von 10 mbar, einem Sumpfvakuum von 25 mbar und einem Rücklaufverhältnis von 2 wurden 502 kg/h eines Kopfproduktes K1 und 1498 kg/h eines Sumpfproduktes S1 (Siedepunkt 219,2 °C) erhalten. Das Sumpfprodukt wurde mit einem Durchsatz von 1498 kg/h bei einer Temperatur von 219,2 °C zwischen Auftriebs- und Abtriebsteil einer zweiten Fraktionierkolonne mit gepackten Einbauten aufgegeben. Bei einem Kopfvakuum von 10 mbar, einem Sumpfvakuum von 25 mbar und einem Rücklaufverhältnis von 10 wurden 150 kg/h eines Kopfproduktes K2 und 1348 kg/h eines Sumpfproduktes S2 (Siedepunkt 219,7 °C) erhalten. Das Sumpfprodukt wurde mit einem Durchsatz von 1348 kg/h bei einer Temperatur von 219,7 °C zwischen Auftriebs- und Abtriebsteil einer dritten Fraktionierkolonne mit gepackten Einbauten aufgegeben. Bei einem Kopfvakuum von 10 mbar, einem Sumpfvakuum von 25 mbar und einem Rücklaufverhältnis von 5,5 wurden 582 kg/h eines Kopfproduktes K3 und 766 kg/h eines Sumpfproduktes S3 (Siedepunkt 229,5 °C) erhalten. Die Ausbeute, definiert als der Quotient aus Fertigprodukt (ungesättigter Methylester) und Feed (Einsatzstoff) betrug 53,1 %. Die Zusammensetzungen der Produkte ist Tabelle 1 zu entnehmen.

**Tabelle 1**

| **Zusammensetzung der Methylesterfraktionen (Angaben als Gew.-%)** | | | | | | | |
|---|---|---|---|---|---|---|---|
| | **PKFSME** | **K1** | **S1** | **K2** | **S2** | **K3** | **S3** |
| C16:0 ME | 25,12 | 96,15 | 1,32 | **13,03** | 0,01 | **0,03** | 0 |
| C18:2 ME | 10,08 | 0,87 | 13,17 | **18,24** | 12,60 | **18,28** | 8,29 |
| C18:1 ME | 55,73 | 2,91 | 73,43 | **66,84** | 74,17 | **80,12** | 69,64 |
| C18:0 ME | 9,07 | 0,07 | 12,09 | **1,89** | 13,22 | **1,57** | 22,07 |

**Beispiel 2.** Palmkemfettsäuremethylesterfraktion (PKFSME 16-18) wurde mit einem Durchsatz von 830 kg/h auf 180 °C erhitzt und kontinuierlich zwischen Auftriebs- und Abtriebsteil einer ersten Fraktionierkolonne mit gepackten Einbauten aufgegeben. Bei einem Kopfvakuum von 10 mbar, einem Sumpfvakuum von 25 mbar und einem Rücklaufverhältnis von 2,5 wurden 208 kg/h eines Kopfproduktes K1 und 622 kg/h eines Sumpfproduktes S1 (Siedepunkt 219,5 °C) erhalten. Das Sumpfprodukt wurde mit einem Durchsatz von 622 kg/h bei einer Temperatur von 219,5 °C zwischen Auftriebs- und Abtriebsteil einer zweiten Fraktionierkolonne mit gepackten Einbauten aufgegeben. Bei einem Kopfvakuum von 10 mbar, einem Sumpfvakuum von 25 mbar und einem Rücklaufverhältnis von 10 wurden 152 kg/h eines Kopfproduktes K2 und 470 kg/h eines Sumpfproduktes S2 (Siedepunkt 219,8 °C) erhalten. Das Sumpfprodukt wurde mit einem Durchsatz von 470 kg/h bei einer Temperatur von 219,8 °C zwischen Auftriebs- und Abtriebsteil einer dritten Fraktionierkolonne mit gepackten Einbauten aufgegeben. Bei einem Kopfvakuum von 10 mbar, einem Sumpfvakuum von 25 mbar und einem Rücklaufverhältnis von 9 wurden 365 kg/h eines Kopfproduktes K3 und 105 kg/h eines Sumpfproduktes S3 (Siedepunkt 231,4 °C) erhalten. Die Ausbeute, definiert als der Quotient aus Fertigprodukt (ungesättigter Methylester) und Feed (Einsatzstoff) betrug 92,8 %. Die Zusammensetzungen der Produkte ist Tabelle 2 zu entnehmen.

**Tabelle 2**

| **Zusammensetzung der Methylesterfraktionen (Angaben als Gew.-%)** | | | | | | | |
|---|---|---|---|---|---|---|---|
| | **PKFSME** | **K1** | **S1** | **K2** | **S2** | **K3** | **S3** |
| C16:0 ME | 25,12 | 99,32 | 0,31 | **1,26** | 0 | **0** | 0 |
| C18:2 ME | 10,08 | 0,16 | 13,40 | **20,67** | 11,04 | **13,69** | 1,84 |
| C18:1 ME | 55,73 | 0,51 | 74,20 | **76,13** | 73,57 | **84,83** | 34,44 |
| C18:0 ME | 9,07 | 0,01 | 12,10 | **1,94** | 15,39 | **1,48** | 63,72 |

## Patentansprüche

1. Verfahren zur Herstellung von technischen Ölsäuremethylestern mit Anteilen an Stearinsäuremethylester kleiner oder gleich 2 Gew.-% und Anteilen an Palmitinsäuremethylester kleiner oder gleich 5 Gew.-%, **dadurch gekennzeichnet, dass** man das Ausgangsgemisch Palmkernfettsäuremethylester PKFSME C8-18 zunächst fraktioniert destilliert, so dass sich im Kopfprodukt PKFSME C8-14 und im Sumpfprodukt PKFSME C16-18 anreichert,
dass nun die Sumpffraktion PKFSME C16-18 derart verarbeitet wird, dass man
(a) PKFSME C16-18 einer ersten fraktionierten Destillation unterwirft und dabei ein kürzerkettiges Kopfprodukt PKFSME C16 und ein überwiegend längerkettiges und ungesättigtes Sumpfprodukt PKFSME C18 herstellt, und
(b) das so erhaltene erste Sumpfprodukt einer zweiten fraktionierten Destillation unterwirft und dabei ein überwiegend ungesättigtes zweites Kopfprodukt mit erhöhtem Palmitinsäuremethylestergehalt und ein ebenfalls überwiegend ungesättigtes Sumpfprodukt mit minimalem Palmitinsäuremethylestergehalt, wobei das zweite Kopfprodukt mindestens 1 Gew.-% Palmitinsäuremethylester und mindestens 60 Gew.-% Ölsäuremethylester aufweist, und
(c) das so erhaltene zweite Sumpfprodukt einer dritten fraktionierten Destillation unterwirft und dabei ein ölsäuremethylesterreiches und stearinsäuremethylesterarmes drittes Kopfprodukt und ein ölsäuremethylester- und stearinsäuremethylesterreiches Sumpfprodukt herstellt, wobei das dritte Kopfprodukt mindestens 75 Gew.-% Ölsäuremethylester und weniger als 2 Gew.-% Stearinsäuremethylester enthält, und
(d) das Zielprodukt durch Mischen des zweiten Kopfproduktes und des dritten Kopfproduktes herstellt, wobei das Zielprodukt mindestens 75 Gew.-% Ölsäuremethylester, maximal 5 Gew.-% Palmitinsäuremethylester und maximal 2 Gew.-% Stearinsäuremethylester enthält.

2. Verfahren nach Anspruch 1, **dadurch gekennzeichnet, dass** man die erste Fraktionierung bei einer Temperatur im Bereich von 160 bis 200 °C und einem verminderten Druck im Bereich von 10 bis 25 mbar durchführt.

3. Verfahren nach den Ansprüchen 1 und/oder 2, **dadurch gekennzeichnet, dass** man ein erstes Kopfprodukt herstellt, welches mindestens 90 Gew.-% Palmitinsäuremethylester enthält.

4. Verfahren nach mindestens einem der Ansprüche 1 bis 3, **dadurch gekennzeichnet, dass** man ein erstes Sumpfprodukt herstellt, welches mindestens 70 Gew.-% Ölsäuremethylester enthält.

5. Verfahren nach mindestens einem der Ansprüche 1 bis 4, **dadurch gekennzeichnet, dass** man die zweite Fraktionierung bei einer Temperatur im Bereich von 200 bis 230 °C und einem verminderten Druck im Bereich von 10 bis 25 mbar durchführt.

6. Verfahren nach mindestens einem der Ansprüche 1 bis 5, **dadurch gekennzeichnet, dass** man ein zweites Sumpfprodukt herstellt, welches höchstens 1 Gew.-% Palmitinsäuremethylester und mindestens 70 Gew.-% Ölsäuremethylester enthält.

7. Verfahren nach mindestens einem der Ansprüche 1 bis 6, **dadurch gekennzeichnet, dass** man die dritte Fraktionierung bei einer Temperatur im Bereich von 210 bis 230 °C und einem verminderten Druck von 10 bis 25 mbar durchführt.

8. Verfahren nach mindestens einem der Ansprüche 1 bis 7, **dadurch gekennzeichnet, dass** man ein drittes Sumpfprodukt herstellt, welches höchstens 30 Gew.-% Ölsäuremethylester und mindestens 20 Gew.-% Stearinsäuremethylester enthält.

## Claims

1. A process for the production of technical oleic acid methyl esters with stearic acid methyl ester contents of or below 2% by weight and palmitic acid methyl ester contents of or below 5% by weight, **characterized in that** the starting mixture, palm kernel fatty acid methyl ester PKFAME C8-18, is first subjected to fractional distillation so that PKFAME C8-14 accumulates in the head product and PKFAME C16-18 accumulates in the bottom product, the bottom fraction PKFAME C16-18 is then processed in such a way that
(a) PKFAME C16-18 is subjected to a first fractional distillation to form a relatively short-chain head product PKFAME C16 and a predominantly relatively long-chain and unsaturated bottom product PKFAME C 18 and
(b) the first bottom product thus obtained is subjected to a second fractional distillation to form a predominantly unsaturated second head product with a high palmitic acid methyl ester content and a likewise predominantly unsaturated bottom product with a minimal palmitic acid methyl ester content, the second head product containing at least 1% by weight of palmitic acid methyl ester and at least 60% by weight of oleic acid methyl ester, and
(c) the second bottom product thus obtained is subjected to a third fractional distillation to form a third head product rich in oleic acid methyl ester and poor in stearic acid methyl ester and a bottom product rich in oleic acid methyl ester and stearic acid methyl ester, the third head product containing at least 75% by weight of oleic acid methyl ester and less than 2% by weight of stearic acid methyl ester, and
(d) the target product is prepared by mixing the second head product and the third head product, the target product containing at least 75% by weight of oleic acid methyl ester, at most 5% by weight of palmitic acid methyl ester and at most 2% by weight of stearic acid methyl ester.

2. A process as claimed in claim 1, **characterized in that** the first fractionation is carried out at a temperature in the range of from 160 to 200°C and under a reduced pressure in the range of from 10 to 25 mbar.

3. A process as claimed in claims 1 and/or 2, **characterized in that** a first head product containing at least 90% by weight of palmitic acid methyl ester is prepared.

4. A process as claimed in at least one of claims 1 to 3, **characterized in that** a first bottom product containing at least 70% by weight of oleic acid methyl ester is prepared.

5. A process as claimed in at least one of claims 1 to 4, **characterized in that** the second fractionation is carried out at a temperature in the range of from 200 to 230°C and under a reduced pressure in the range of from 10 to 25 mbar.

6. A process as claimed in at least one of claims 1 to 5, **characterized in that** a second bottom product containing at most 1% by weight of palmitic acid methyl ester and at least 70% by weight of oleic acid methyl ester is prepared.

7. A process as claimed in at least one of claims 1 to 6, **characterized in that** the third fractionation is carried out at a temperature in the range of from 210 to 230°C and under a reduced pressure of 10 to 25 mbar.

8. A process as claimed in at least one of claims 1 to 7, **characterized in that** a third bottom product containing at most 30% by weight of oleic acid methyl ester and at least 20% by weight of stearic acid methyl ester is prepared.

## Revendications

1. Procédé pour la préparation d'esters méthyliques de l'acide oléique techniques avec des proportions d'ester méthylique de l'acide stéarique inférieures ou égales à 2% en poids et des proportions d'ester méthylique de l'acide palmitique inférieures ou égales à 5% en poids, **caractérisé**
**en ce qu'**on distille de manière fractionnée d'abord le mélange de départ d'esters méthyliques des acides gras de noyau de palme PKFSME C8-18 de manière telle que le PKFSME C8-14 s'enrichit dans le produit de tête et le PKFSME C16-18 dans le produit de fond,
**en ce qu'**à présent, la fraction de fond PKFSME C16-18 est transformée en ce qu'on
(a) soumet le PKFSME C16-18 à une première distillation fractionnée et on prépare un produit de tête PKFSME C16 à courte chaîne et un produit de fond PKFSME C18 principalement à longue chaîne et insaturé, et
(b) on soumet le premier produit de fond ainsi obtenu à une deuxième distillation fractionnée et on obtient ainsi un deuxième produit de tête principalement insaturé présentant une teneur augmentée en ester méthylique de l'acide palmitique et un produit de fond également principalement insaturé présentant une teneur minimale en ester méthylique de l'acide palmitique, le deuxième produit de tête présentant au moins 1% en poids d'ester méthylique de l'acide palmitique et au moins 60% en poids d'ester méthylique de l'acide oléique, et
(c) on soumet le deuxième produit de fond ainsi obtenu à une troisième distillation fractionnée et on obtient ainsi un troisième produit de tête riche en ester méthylique de l'acide oléique et pauvre en ester méthylique de l'acide stéarique et un produit de fond riche en ester méthylique de l'acide oléique et riche en ester méthylique de l'acide stéarique, le troisième produit de tête contenant au moins 75% en poids d'ester méthylique de l'acide oléique et moins de 2% en poids d'ester méthylique de l'acide stéarique, et
(d) on prépare le produit cible par mélange du deuxième produit de tête et du troisième produit de tête, le produit cible contenant au moins 75% en poids d'ester méthylique de l'acide oléique, au maximum 5% en poids d'ester méthylique de l'acide palmitique et au maximum 2% en poids d'ester méthylique de l'acide stéarique.

2. Procédé selon la revendication 1, **caractérisé en ce qu'**on réalise le premier fractionnement à une température dans la plage de 160 à 200°C et à une pression réduite dans la plage de 10 à 25 mbars.

3. Procédé selon les revendications 1 et/ou 2, **caractérisé en ce qu'**on prépare un premier produit de tête qui contient au moins 90% en poids d'ester méthylique de l'acide palmitique.

4. Procédé selon au moins l'une quelconque des revendications 1 à 3, **caractérisé en ce qu'**on prépare un premier produit de fond qui contient au moins 70% en poids d'ester méthylique de l'acide oléique.

5. Procédé selon au moins l'une quelconque des revendications 1 à 4, **caractérisé en ce qu'**on réalise le deuxième fractionnement à une température dans la plage de 200 à 230°C et à une pression réduite dans la plage de 10 à 25 mbars.

6. Procédé selon au moins l'une quelconque des revendications 1 à 5, **caractérisé en ce qu'**on prépare un deuxième produit de fond qui contient au plus 1% en poids d'ester méthylique de l'acide palmitique et au moins 70% en poids d'ester méthylique de l'acide oléique.

7. Procédé selon au moins l'une quelconque des revendications 1 à 6, **caractérisé en ce qu'**on réalise le troisième fractionnement à une température dans la plage de 210 à 230°C et à une pression réduite dans la plage de 10 à 25 mbars.

8. Procédé selon au moins l'une quelconque des revendications 1 à 7, **caractérisé en ce qu'**on prépare un troisième produit de fond qui contient au plus 30% en poids d'ester méthylique de l'acide oléique et au moins 20% en poids d'ester méthylique de l'acide stéarique.
